# EUROPÄISCHE PATENTSCHRIFT

(11) **EP 2 977 740 B1**
(45) Veröffentlichungstag und Bekanntmachung des Hinweises auf die Patenterteilung: **30.05.2018**
(21) Anmeldenummer: 14177844.9
(22) Anmeldetag: 21.07.2014
(51) Int. Cl.: G01N 1/04, G01N 33/36

(54) **Vorrichtung sowie Verfahren zum automatischen Entnehmen von Einzelfasern aus einem Faserspeicher und zum Überführen einer Einzelfaser zu einem nachfolgenden Gerät**
Device and method for automatic sampling of individual fibres from a fibre reservoir and for the transfer of an individual fibre to a subsequent device
Dispositif et procédé pour l'échantillonnage automatique de fibres singulaire d'un réservoir de fibres et pour transférer une fibre singulaire à un appareil subséquent

(43) Veröffentlichungstag der Anmeldung: 27.01.2016
(73) Patentinhaber: TEXTECHNO HERBERT STEIN GMBH & CO. KG, 41066 Mönchengladbach (DE)
(72) Erfinder: Mörschel, Ulrich, 52441 Linnich (DE)
(74) Vertreter: dompatent von Kreisler Selting Werner - Partnerschaft von Patent- und Rechtsanwälten mbB

(56) Entgegenhaltungen:
- DE-A1- 3 836 826
- DE-T2- 69 916 998
- US-A- 6 085 584
- US-A1- 2002 178 547
- US-A1- 2004 141 188

## Beschreibung

Die Erfindung betrifft eine Vorrichtung und ein Verfahren zum automatischen Entnehmen von Einzelfasern einer Faserprobe aus einem Faserspeicher und zum Überführen einer Einzelfaser zu einem nachfolgenden Gerät, insbesondere einem Faserprüfgerät, nach dem Oberbegriff des Anspruchs 1 bzw. 8.

Aus der DE 699 16 998 T2 sind ein Verfahren und eine Vorrichtung zur Verarbeitung von faserigem Material wie Baumwolle, insbesondere zur Entkörnung von Baumwolle bekannt. Zur Optimierung des entnommenen faserigen Materials durch Anpassung der Prozessparameter weist die Vorrichtung eine Einrichtung zur Entnahme einer physischen Probe aus dem Entkörnungsprozessstrom auf. Die Baumwollproben, welche eine Vielzahl von Einzelfasern aufweisen, werden dabei über eine Entnahmeeinrichtung in Form von rotierenden Kardierwalzen oder alternativ in Form von Kämmen auf einem umlaufenden Band entnommen und anschließend im Hinblick auf charakteristische Eigenschaften analysiert.

Eine Vorrichtung zur Prüfung von Baumwollfasern ist aus der US 2002/0178547 A1 bekannt, bei welcher Faserproben ebenfalls mittels einer rotierenden Kardierwalze entnommen werden.

Aus der US 6,085,584 A ist eine weitere Vorrichtung zur automatisierten Faserprüfung bekannt, bei welcher Faserproben mittels eines beweglichen Nadelkamms aus einem Faserballen entnommen werden. Die aus einer Vielzahl von Einzelfasern bestehende Faserprobe kann in dem Nadelkamm mittels einer Einspannvorrichtung eingeklemmt werden, um darauffolgend mechanische Eigenschaften der Faserproben zu testen.

Aus der US 2004/0141188 A1 sind eine Vorrichtung und ein Verfahren zur Kontrolle eines kontinuierlichen Stroms von Einzelfasern zur Bestimmung von Faserlängen bekannt. Dabei wird ein luftgebundener Einzelfaserstrom in ein elektrostatisches Feld zwischen zwei Elektrodenplatten mit einem Laufband eingebracht. Aufgrund der elektrostatischen Ladung haften die Einzelfasern mit einem Ende an dem Laufband und werden gleichzeitig in Faserlängsrichtung zwischen den Elektrodenplatten gestreckt. Die gestreckten Fasern werden mittels des Laufbands an einem Sensor zur Bestimmung der Faserlänge vorbeigeführt.

Eine lanzenförmige Entnahmevorrichtung von Materialproben aus gepressten Faserballen ist aus der DE 3836826 A1 bekannt.

Stapelfasern sind Fasern begrenzter Länge, z.B. aus Baumwolle, Wolle und Humanhaar, Viskose, Polyester, Acryl oder anderen textilen oder technischen Fasermaterialien. Im textilen Prüfwesen werden bei Chemiefasern einschließlich der Viskose die meisten Eigenschaften solcher Fasern an Einzelfasern bestimmt. Dazu zählt beispielsweise die Messung von Reißfestigkeit und Dehnung, Länge, Feinheit, Kräuselung, Schrumpf oder Reibung. In der Regel wird für solche Prüfungen eine Einzelfaser nach der anderen manuell mittels Pinzette aus der Faserprobe, meist in Form eines Faserbausches oder -bündels, entnommen und anschließend in das entsprechende Prüfgerät eingeführt. Dieses Verfahren ist zeit- und arbeitsintensiv, insbesondere da zur Erzielung statistisch gesicherter Ergebnisse immer eine größere Zahl von Einzelprüfungen notwendig ist.

Eine Vorrichtung zum automatischen Entnehmen einer Einzelfaser aus einem Magazin ist beispielsweise aus der DE 43 43 157 C1 bekannt. Diese Vorrichtung umfasst ein Magazin zur Aufnahme von Einzelfasern einer Stapelfaserprobe, die in einem Rahmen mit mehreren Klemmen zur Befestigung jeweils einer Einzelfaser gehalten sind.

Die Vorrichtung ist kombiniert mit einem Prüfgerät zur Charakterisierung von mechanischen und/oder geometrischen Eigenschaften der Stapelfaserproben in dem mindestens eine obere vertikal und horizontal bewegbare Klemmbacke des Prüfgerätes zum Halten und Transportieren einer zu charakterisierenden Stapelfaserprobe dient, die lösbar in dem Magazin gehalten ist.

Es ist ferner eine Transportvorrichtung zum Bewegen der Klemmen des Magazins sowie eine Justiervorrichtung zum Positionieren einer Klemme des Magazins in der Nähe der oberen Klemmbacke vorgesehen.

Die Transportvorrichtung bewegt die obere Klemmbacke des Prüfgerätes horizontal aus einer Messposition in eine Übernahmeposition und wieder zurück.

Die aus der DE 43 43 157 C1 bekannte Vorrichtung ist zeit- und arbeitsintensiv, da die Einzelfasern manuell vereinzelt und dann zunächst in einen Rahmen des Fasermagazins an einer Vielzahl von Klemmen befestigt werden müssen, wobei außerdem die Anzahl der in dem Magazin gehaltenen Stapelfaserproben sehr begrenzt ist.

Bei einem anderen automatischen Gerät zur Prüfung von Festigkeitseigenschaften und Faserdicke von Einzelfasern werden ebenfalls Einzelfasern in einem Magazin vorbereitet und danach automatisch entnommen und dem Prüfgerät zugeführt. Die Vorbereitung ist hier allerdings relativ aufwändig, da an den Enden der Einzelfasern Klemmstücke befestigt werden müssen. Außerdem ist die Magazinkapazität mit maximal 100 Einzelfasern begrenzt.

Eine andere Form der Rationalisierung bietet eine weitere Vorrichtung zum automatischen Überführen der Einzelfasern in Verbindung mit einem Prüfgerät (FAVIMAT-ROBOT der Firma Textechno). Diese Vorrichtung beinhaltet beispielsweise einen Faserspeicher mit 20 Magazinen für je z.B. 25 Einzelfasern und eine Transporteinrichtung, die eine Einzelfaser nach der anderen aus den Magazinen entnimmt und dem Prüfgerät zuführt. Angesichts der Speicherkapazität von maximal 500 Einzelfasern erlaubt das System einen vollautomatischen Prüfablauf über große Zeiträume. Lediglich das Befüllen der Magazine erfolgt hier noch manuell. Bei Kombinationen der verschiedenen, mit dem Prüfgerät durchführbaren Prüfverfahren kann die Gesamtdauer der Prüfung an 500 Fasern mehr als 30 Stunden betragen. Demgegenüber dauert das Einbringen der 500 Fasern in 20 Magazine nach entsprechender Einarbeitung der Bedienungsperson ca. eine Stunde.

Der Erfindung liegt die Aufgabe zugrunde, eine Vorrichtung und ein Verfahren zum automatischen Entnehmen von Einzelfasern einer Faserprobe aus einem Faserspeicher und zum Überführen der Einzelfasern zu einem nachfolgenden Gerät, insbesondere einem Faserprüfgerät, derart weiterzubilden, dass ein vollautomatischer, ununterbrochener Probenahmeablauf über große Zeiträume möglich ist, und außerdem die manuelle Befüllung von Magazinen mit Einzelfasern vermieden wird.

Zur Lösung dieser Aufgabe dienen die Merkmale der Ansprüche 1 bzw. 8.

Die Erfindung sieht in vorteilhafter Weise vor, dass der Faserspeicher mindestens einen Probenbehälter aufweist, in dem die Faserprobe, z.B. in Form von Faserbüscheln, enthalten ist, dass eine Entnahmeeinrichtung eine oder mehrere Einzelfasern aus einem in dem Probenbehälter enthaltenen Faserbüschel entnimmt, die entnommenen Einzelfasern vereinzelt, dass die Transportvorrichtung die vereinzelte Einzelfaser von der Entnahmevorrichtung zur Weiterführung der Einzelfaser an das nachfolgende Gerät übernimmt und dass die Faservereinzelungseinrichtung die entnommenen Einzelfasern der Greifeinrichtung streckt bis nur eine einzige Einzelfaser detektiert wird und dann die vereinzelte Einzelfaser an die Transporteinrichtung, beispielsweise an eine Klemmeinrichtung, z.B. eine Klemmzange, der Transporteinrichtung freigibt.

Das nachfolgende Gerät ist vorzugsweise ein Faserprüfgerät, kann aber auch z.B. ein Gerät zur chemischen Behandlung der Einzelfaser oder ein Gerät sein, das aus Einzelfasern eine Verbundstruktur erzeugt.

Die Erfindung ermöglicht es somit, auch den Vereinzelungsvorgang zu automatisieren, wodurch der Zeitaufwand für die Vereinzelung der Einzelfasern der Probe erheblich reduziert werden kann.

Obwohl beim Stand der Technik der Arbeitsaufwand für die Einführung der Fasern in die Magazine in Relation zur Laufzeit des nachfolgenden Geräts, z. B. eines Prüfgerätes, gering ist, erscheint es sinnvoll, auch diesen Aufwand zu eliminieren. Die Faserprobe, der Faserbausch oder das Faserbündel, bei denen sich die Einzelfasern mehr oder weniger in Wirrlage befinden, wird in einen topfförmigen Behälter eingebracht. Aus diesem zieht eine Greifeinrichtung zunächst eine kleine Gruppe von Fasern heraus, vereinzelt diese und übergibt anschließend eine letzte verbleibende Einzelfaser an die Transporteinrichtung, die die Einzelfaser an das nachfolgende Gerät überführt.

Vorzugsweise ist vorgesehen, dass die Entnahmeeinrichtung zumindest eine Greifeinrichtung und eine Faservereinzelungseinrichtung aufweist, wobei die Greifeinrichtung eine oder mehrere Einzelfasern aus dem Probenbehälter entnimmt.

Die Greifeinrichtung ist vorzugsweise an der Spitze kegelförmig und nahezu nadelförmig dünn, wobei beim Greifvorgang möglichst wenig Einzelfasern aus dem Faserbüschel in dem Probenbehälter erfasst werden sollen.

Bei einer bevorzugten Ausführungsform ist vorgesehen, dass die Greifeinrichtung, insbesondere eine Greiferklemme, die von der Greifeinrichtung entnommenen und gehaltenen Einzelfasern durch mehrere Verengungen der Faservereinzelungseinrichtung zieht, in denen die Einzelfasern in eine gestreckte Form überführbar sind. Aufgrund der unterschiedlichen Längen der Einzelfaserenden im Klemmzustand ist es möglich, die Faservereinzelung solange zu betätigen, bis sich nur noch eine Einzelfaser zwischen den Verengungen befindet. Die Einzelfasern können dabei, auch wie bei Chemiefasern üblich, alle die gleiche Stapellänge aufweisen.

Hierzu ist vorzugsweise vorgesehen, dass die Entnahmeeinrichtung eine Überwachungseinrichtung aufweist, die die Anzahl der gestreckten Einzelfasern im Übergabebereich detektiert, wobei z.B. eine Klemmzange der Transporteinrichtung zur Übernahme der vereinzelten Einzelfaser von der Entnahmeeinrichtung aktivierbar ist, wenn nur noch eine einzige Einzelfaser in einem überwachten Übergabebereich für die Transporteinrichtung vorhanden ist.

Die Detektion der Einzelfaser erfolgt dabei vorzugsweise optisch.

Der Faserspeicher besteht bei einer Weiterbildung der Erfindung aus einem Magazin mit mehreren Probenbehältern mit gleichen oder unterschiedlichen Faserproben. Der Faserspeicher ist vorzugsweise auf einem Karussell angeordnet, so dass jeder Probenbehälter in eine bestimmte vorgegebene Probeentnahmeposition verfahrbar ist.

In dieser Probeentnahmeposition kann mindestens eine Greifeinrichtung durch mindestens eine Öffnung in ein Faserbüschel in den Probenbehälter hineingefahren werden, wobei durch Betätigung der Greifeinrichtung eine oder mehrere Einzelfasern aus dem Faserbüschel entnehmbar sind und aus dem Probenbehälter herausziehbar sind.

Es kann auch vorgesehen sein, dass der mindestens eine Probenbehälter mehrere Öffnungen für den Durchtritt der mindestens einen Greifeinrichtung aufweist. Auf diese Weise können Proben an unterschiedlichen Stellen entnommen werden oder auch mehrere Greifeinrichtungen zum Einsatz kommen.

Die Verengungen sind vorzugsweise durch jeweils zwei mit einander angepassten Aussparungen versehene Schwenkarmpaare gebildet, deren Schwenkstellung und damit die Größe der Verengung zwischen den Schwenkarmpaaren durch die Steuerung einstellbar ist. Die durch die Aussparungen gebildeten Verengungen können in ihrem effektiven Durchmesser mit Hilfe einer entsprechenden Positionierung der Schwenkarmpaare verändert werden. Es versteht sich, dass die Verengung auch mit einem einzigen Schwenkarm und einem starren Arm gebildet werden kann.

Die Faserbüschel sind in dem mindestens einen Probenbehälter beispielsweise durch ein aufliegendes Gewicht komprimierbar.

Bei einer Weiterbildung der Erfindung kann vorgesehen sein, dass die Faserbüschel in dem mindestens einen Probenbehälter über eine an der mindestens einen Öffnung für den Durchtritt der mindestens einen Greifeinrichtung angeordneten Blasdüse mit Pressluft durchmischbar sind.

Im Folgenden wird unter Bezugnahme auf die Zeichnungen ein Ausführungsbeispiel der Erfindung näher erläutert.

Es zeigen:
- Fig. 1: eine Kombination eines Prüfgerätes mit einer Vorrichtung zur automatischen Entnahme von Einzelfasern aus einem Fasermagazin und Überführen der Einzelfasern zu dem Prüfgerät,
- Fig. 2: ein Fasermagazin mit mehreren Probenbehältern und einer Entnahmeeinrichtung mit integrierter Greifeinrichtung und Vereinzelungseinrichtung,
- Fig. 3: die Entnahmeeinrichtung in vergrößerter Darstellung,
- Fig. 4: das Zusammenwirken der Greifeinrichtung und der Vereinzelungseinrichtung,
- Fig. 5: die Vereinzelung der entnommenen Einzelfasern, und
- Fig. 6: die Übergabe an eine Klemmzange der Transporteinrichtung.

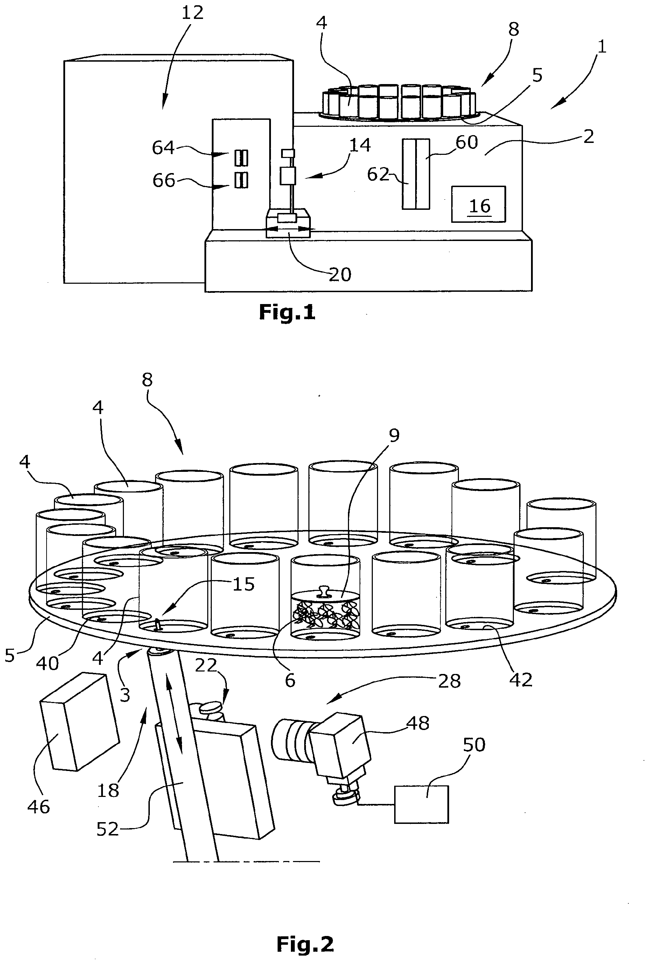

Fig. 1 zeigt eine Vorrichtung 1 zum automatischen Entnehmen von Einzelfasern 10 aus einem Faserspeicher 8 und zum Überführen einer Einzelfaser zu einem nachfolgenden Gerät, z. B. einem in Fig. 1 gezeigten Faserprüfgerät 12, die auch bei bereits vorhandenen Faserprüfgeräten nachgerüstet werden kann.

Das Faserprüfgerät 12 besteht beispielsweise aus einem Prüfgerät wie es unter der Bezeichnung "FAVIMAT +" der Firma Textechno bekannt ist.

In Fig. 1 ist die zwischen der Vorrichtung zur Entnahme von Einzelfasern 10 und dem Faserprüfgerät 12 hin- und herbewegbare Transporteinrichtung 14 ersichtlich, die auf einem Schlitten 20 eine einzelne entnommene Einzelfasern 10 von einer Öffnung 60 im Gehäuse 2 der Vorrichtung 1 zu einem Klemmenpaar 64, 66 des Faserprüfgerätes 12 transportiert.

Die Transporteinrichtung 14 mit der Klemmzange 13 kann seitlich zwischen den Geräten 1 und 12 hin- und herbewegt werden kann und in Richtung auf die Geräte zu und zurück. Die Öffnung 60 im Gehäuse 2 kann z.B. mit einer zweiflügeligen Klapptür 62 versehen sein, um einerseits einen Lichteinfall und andererseits um eine Luftbewegung zu verhindern.

Die Vorrichtung 1 zum automatischen Entnehmen von Einzelfasern weist ein Gehäuse 2 auf, auf dessen Oberseite sich ein Faserspeicher 8 befindet. Der Faserspeicher 8 weist eine Vielzahl von Probenbehältern 4 auf, die vorzugsweise kreisförmig auf einem karussellartigen Magazinteller 5 angeordnet sind, welcher im Bereich der Probenbehälter 4 jeweils im Boden des Magazintellers 5 eine Öffnung 40 für den Durchtritt einer Greifeinrichtung 3 von unten aufweist.

Der Magazinteller 5 weist der Behälterquerschnittsform angepasste Aussparungen 42 auf, in die die Probenbehälter 4 einsetzbar oder einklebbar sind. Der Magazinteller 5 kann beispielsweise 20 Probenbehälter 4 aufnehmen. Eine Faserprobe 6 kann in Form eines Faserbausches oder Faserbündels oder Faserbüschels in die Probenbehälter 4 eingegeben werden. Innerhalb der Aussparung 42 ist die Öffnung 40 vorgesehen.

Die Probenbehälter 4 bestehen vorzugsweise aus einem nach unten offenen Rohrteil, das in die Aussparungen 42 eingesetzt oder eingeklebt werden kann.

Werden nach unten geschlossene Probenbehälter eingesetzt, weisen diese selbst jeweils eine Öffnung in ihrem Boden auf, die dazu dient, dass die Greifeinrichtung 3 von unten durch die Öffnung 40 des Magazintellers 5 in den Probenbehälter 4 hineingreifen kann.

Da die Fasern nicht manuell vereinzelt werden müssen, ist die Befüllung der Probenbehälter 4 sehr schnell durchführbar. Die Probenbehälter 4 können jeweils unterschiedliche Faserqualitäten enthalten.

Die in den Probenbehältern 4 befindlichen Faserproben 6 können mit einem Deckel 9 gewichtsbelastet werden, so dass sie leicht komprimiert werden.

Eine Steuerung 16, die mit dem Faserprüfgerät 12 kooperiert oder in dieses Faserprüfgerät 12 integriert ist, übernimmt die Steuerung der automatischen Probeentnahme. Hierzu wird in die Steuerung 16 eingegeben, welche Faserprobe 6 sich in den einzelnen Probenbehältern 4 auf dem Karussell 5 befindet. Die Steuerung 16 übernimmt die Auswahl des Probenbehälters 4 für die Probenahme und bringt die entsprechende Öffnung 40 in eine Position, in der eine Greifeinrichtung 3 durch die Öffnung 40 in den ausgewählten Probenbehälters 4 hindurchtreten kann.

Die Greifeinrichtung 3 ist Bestandteil einer Entnahmeeinrichtung 18, die außer der Greifeinrichtung 3 eine Faservereinzelungseinrichtung 22, eine Transporteinrichtung 14 und ein optisches Detektionssystem 28, bestehend aus einer Kamera 48, einer Lichtquelle 46 und einer Bildverarbeitungseinrichtung 50, aufweist.

Die Greifeinrichtung 3 weist einen motorisch auf- und abbewegbaren Hubkörper 52 auf. An der Spitze des Hubkörpers 52 ist eine lanzenförmige KolbenzylinderEinheit 54 angeordnet, mit einem zylindrischen rohrförmigen Teil 56, aus dem eine Kolbenstange 58 mit kegelförmiger Spitze herausfahrbar ist, um eine Klemmeinrichtung 15 für Einzelfasern 10 zu bilden.

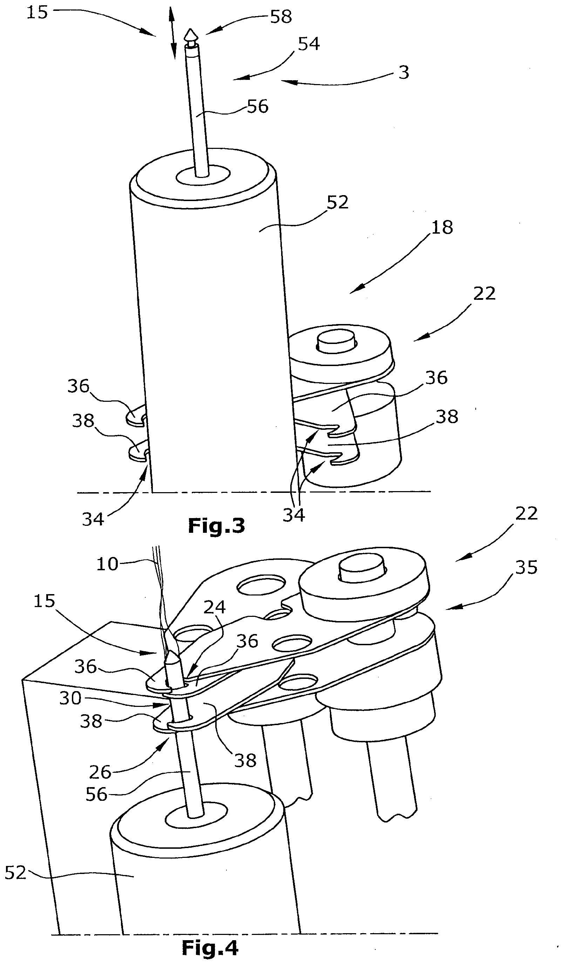

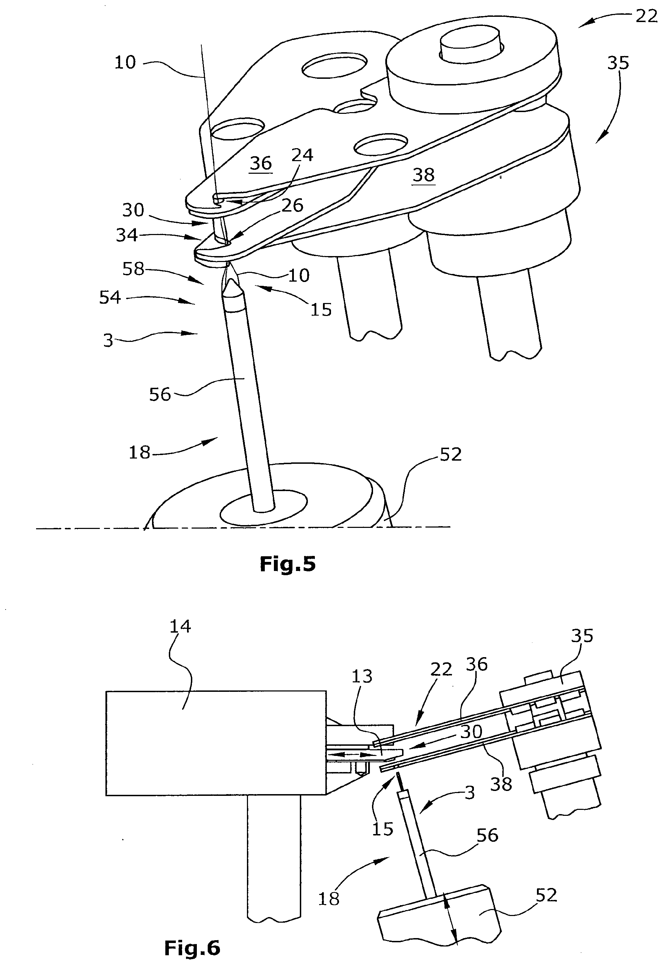

Fig. 3 zeigt die Klemmeinrichtung 15 in geöffnetem Zustand, während die Fign. 4 und 5 die Klemmeinrichtung 15 in geschlossenem Zustand zeigen.

Es versteht sich, dass die Klemmeinrichtung 15 für die Einzelfasern 10 auch anders gestaltet sein kann, z.B. in Form einer aus zwei Drahtwinkeln bestehenden Klemmzange. Eine weitere Alternative besteht insbesondere zur Anwendung bei gekräuselten Fasern aus einem spitzen Haken.

Für die Probeentnahme wird daher der Hubkörper 52 nach oben bewegt, so dass die Klemmeinrichtung 15 durch die Öffnung 40 in die Unterseite des Probenbehälters 4 eingeführt werden kann. Die Kolbenstange 58 mit der kegelförmigen Spitze befindet sich im Öffnungszustand und wird in die Faserprobe 6 hineingestoßen. Beim Schließen der Klemmeinrichtung 15 durch Zurückziehen der Kolbenstange 58 werden eine oder mehrere Einzelfasern 10 eingeklemmt, so dass sie durch Herunterfahren des Hubkörpers 52 aus dem Probenbehälter 4 entnommen werden können.

Falls die Klemmeinrichtung 15 keine Einzelfaser 10 greifen konnte, und dies in einem Übergabebereich 30 mit Hilfe des Detektionssystems 28 detektiert wird, veranlasst die Steuerung 16 eine Wiederholung des Entnahmevorgangs.

Die Kolbenstange 58 der Klemmeinrichtung 15 wird vorzugsweise pneumatisch bewegt.

Die von der Klemmeinrichtung 15 gehaltenen Einzelfasern 10 werden durch die Rückzugsbewegung des Hubkörpers 52 einer Faservereinzelungseinrichtung 22 zugeführt. Die Faservereinzelungseinrichtung 22 bildet zwei Verengungen 24, 26, durch die die Klemmeinrichtung 15 mit den von der Klemmeinrichtung 15 gehaltenen Einzelfasern 10, wie aus den Fign. 4 und 5 ersichtlich, hindurchgezogen wird.

Die Verengungen 24, 26 werden jeweils durch Schwenkarme gebildet, wobei ein erstes Schwenkarmpaar 36 die erste Verengungen 24 bildet und ein zweites Schwenkarmpaar 38 die zweite Verengung 26 mit Abstand unter der ersten Verengung 24 bildet. Die Verengungen 24, 26 werden dabei mit Hilfe von einander angepassten Aussparungen 34 in den jeweiligen Schwenkarmen der Schwenkarmpaare 36, 38 gebildet.

Die Schwenkarmpaare 36, 38 können so weit geöffnet werden, dass auch der Hubkörper 52 durch sie hindurchtreten kann, wie aus Fig. 3 ersichtlich ist.

Zum Vereinzeln der von der Klemmeinrichtung 15 erfassten Einzelfasern 10 werden die Schwenkarmpaare 36, 38 geschlossen, wie dies aus Fig. 4 ersichtlich ist. Ist die Klemmeinrichtung 15 so weit heruntergefahren, dass sie die Schwenkarmpaare 36, 38 passiert hat, schließen sich die Schwenkarmpaare 36, 38 weiter, so dass engere Verengungen 24 und 26 gebildet werden. Die Steuerung 16, die den gesamten Bewegungsablauf steuert, kann dabei Verengungen 24, 26 unterschiedlichen Durchmessers erzeugen.

Die im Wesentlichen aus der Kamera 48 und der Bildverarbeitungseinrichtung 50 bestehende Detektionseinrichtung 28 überwacht den Übergabebereich 30 zwischen den Schwenkarmpaaren 36, 38 und detektiert, wann sich nur noch eine Einzelfaser 10 im Übergabebereich 30 zwischen der ersten und zweiten Verengung 24, 26 befindet.

Wird nur noch eine Einzelfaser 10 in dem Übergabebereich 30 festgestellt, wird eine Transporteinrichtung 14 aktiviert, die die Einzelfaser 10 z.B. mit einer Klemmzange 13 aus dem Übergabebereich 30 übernimmt und die Einzelfaser 10 zu dem Faserprüfgerät 12 transportiert.

Die Faservereinzelungseinrichtung 22 befindet sich demzufolge in diesem Beispiel unterhalb des Faserspeichers 8. Die Schwenkarmpaare 36, 38 sind motorisch durch eine Antriebs- und Getriebeeinheit 35 angetrieben, die die Schwenkarme jedes Schwenkarmpaares 36, 38 motorisch voneinander weggedreht oder mehr oder weniger aufeinander zugedreht.

Die Faservereinzelungseinrichtung besteht nicht nur aus den beiden Schwenkarmpaaren 36, 38, sondern beinhaltet auch die optische Detektion mit der Detektionseinrichtung 28. Die Faservereinzelung arbeitet in Verbindung mit diesen Komponenten und mit der Bewegung der Entnahmeeinrichtung 18.

## Patentansprüche

1. Vorrichtung (1) zum automatischen Entnehmen von Einzelfasern (10) einer Faserprobe (6) aus einem Faserspeicher (8) und zum Überführen einer Einzelfaser (10) zu einem nachfolgenden Gerät, insbesondere einem Faserprüfgerät (12), mit einer Steuerung (16) und einer Transporteinrichtung (14) für die Einzelfasern (10), umfassend
einen Faserspeicher (8), welcher mindestens einen Probenbehälter (4) aufweist, in dem die Faserprobe (6) in Form von Faserbüscheln enthalten ist,
eine Entnahmeeinrichtung (18), welche zur Entnahme mehrerer Einzelfasern (10) aus dem in dem Probenbehälter (4) enthaltenen Faserbüschel der Faserprobe (6) eingerichtet ist,
eine Transportvorrichtung (14), die dazu eingerichtet ist, die entnommenen Einzelfasern (10) von der Entnahmeeinrichtung (18) zur Weiterführung der Einzelfasern (10) an das nachfolgende Gerät (12) zu übernehmen,
**dadurch gekennzeichnet, dass**
die Entnahmeeinrichtung (18) zumindest eine Greifeinrichtung (3), eine Faservereinzelungseinrichtung (22) und eine Detektionseinrichtung (28) aufweist, wobei
die Greifeinrichtung (3) eingerichtet ist eine oder mehrere Einzelfasern (10) aus dem Probenbehälter (4) zu entnehmen, und dass die Faservereinzelungseinrichtung (22) eingerichtet ist, die von der Greifeinrichtung (3) entnommenen Einzelfasern (10) zu strecken, bis nur eine einzige Einzelfaser (10) von der Detektionseinrichtung (28) detektiert wird und dann die vereinzelte einzige Einzelfaser (10) an eine Klemmeinrichtung (13) der Transporteinrichtung (14) Freizugeben.

2. Vorrichtung nach Anspruch 1, **dadurch gekennzeichnet, dass** die durch die Greifeinrichtung (3) entnehmbaren und gehaltenen Einzelfasern (10) durch mehrere Verengungen (24,26) der Faservereinzelungseinrichtung (22) ziehbar sind, in denen die Einzelfasern (10) in eine gestreckte Form überführbar sind.

3. Vorrichtung nach einem der Ansprüche 1 bis 2, **dadurch gekennzeichnet, dass** die Anzahl der gestreckten Einzelfasern (10) durch ein Detektionssystem (28) der Entnahmeeinrichtung (18) detektierbar sind, wobei die Transporteinrichtung (14) zur Übernahme der vereinzelten Einzelfaser (10) aktivierbar ist, wenn nur noch eine einzige Einzelfaser (10) in einem Übergabebereich (30) für die Transporteinrichtung (14) vorhanden ist.

4. Vorrichtung nach einem der Ansprüche 1 bis 3, **dadurch gekennzeichnet, dass** der Faserspeicher (8) aus einem Magazin mit mehreren Probenbehältern (4) mit gleichen oder unterschiedlichen Faserproben (6) besteht.

5. Vorrichtung nach einem der Ansprüche 1 bis 4, **dadurch gekennzeichnet, dass** mindestens eine Greifeinrichtung (3) durch mindestens eine Öffnung (40) in die Faserprobe (6) in dem Probenbehälter (4) einfahrbar ist, wobei durch Betätigung der Greifeinrichtung (3) eine oder mehrere Einzelfasern (10) aus der Faserprobe (6) einklemmbar sind und aus dem Probenbehälter (4) herausziehbar sind.

6. Vorrichtung nach einem der Ansprüche 2 bis 5, **dadurch gekennzeichnet, dass** die Verengungen (24, 26) durch zwei mit einander angepassten Aussparungen (34) versehene Schwenkarmpaare (36, 38) gebildet werden, deren Schwenkstellung und damit die Größe der Verengungen (24, 26) zwischen den Schwenkarmpaaren (36, 38) durch die Steuerung (16) einstellbar ist.

7. Vorrichtung nach einem der Ansprüche 1 bis 6, **dadurch gekennzeichnet, dass** die Faserproben (6) in dem mindestens einen Probenbehälter (4) über eine an der mindestens einen Öffnung (40) für den Durchtritt der mindestens einen Greifeinrichtung (3) angeordneten Blasdüse mit Pressluft durchmischbar sind.

8. Verfahren zum automatischen Entnehmen von Einzelfasern (10) einer Faserprobe (6) aus einem Faserspeicher (8) und zum Überführen einer Einzelfaser (10) zu einem nachfolgenden Gerät, insbesondere einem Faserprüfgerät (12), wobei aus einer in einem Faserspeicher (8) mit mindestens einem Probenbehälter (4) in Form von Faserbüscheln enthaltenen Faserprobe (6) mehrere Einzelfasern (10) entnommen werden und die entnommenen Einzelfasern (10) an das nachfolgende Gerät (12) überführt werden, **dadurch gekennzeichnet, dass** mit Hilfe einer Entnahmeeinrichtung (18) eine oder mehrere Einzelfasern (10) aus dem Probenbehälter (4) entnommen werden, die entnommenen Einzelfasern (10) auf eine einzige Einzelfaser (10) reduziert und gestreckt werden, bis nur eine einzige Einzelfaser (10) detektiert wird und dann die vereinzelte Einzelfaser (10) für den Transport zu dem nachfolgenden Gerät (12) freigegeben wird.

9. Verfahren nach Anspruch 8, **dadurch gekennzeichnet, dass** die entnommenen Einzelfasern (10) durch mehrere Verengungen (24, 26) gezogen werden, in denen die entnommenen Einzelfasern (10) in eine gestreckte Form überführt werden, wobei die Anzahl der in einem Übergabebereich (30) für den Transport zum nachfolgenden Gerät (12) befindlichen Einzelfasern (10) detektiert wird, und die Übernahme einer vereinzelten Einzelfaser (10) zum Transport aktiviert wird, wenn nur noch eine einzige Einzelfaser (10) in dem Übergabebereich (30) vorhanden ist.

10. Verfahren nach Anspruch 8 bis 9, **dadurch gekennzeichnet, dass** Einzelfasern (10) der in dem Probenbehälter (4) enthaltenen Faserprobe (6) dadurch entnommen werden, dass eine Greifeinrichtung (3) über mindestens eine Öffnung (40) geöffnet in die Faserprobe (6) hineingefahren wird, innerhalb der Faserprobe (6) geschlossen wird und anschließend mit der Greifeinrichtung (3) Einzelfasern (10) aus der Faserprobe (6) herausgezogen werden.

11. Verfahren nach Anspruch 8 bis 10, **dadurch gekennzeichnet, dass** mehrere Proben an verschiedenen Stellen innerhalb eines Probenbehälters (4) entnommen werden.

12. Verfahren nach Anspruch 8 bis 11, **dadurch gekennzeichnet, dass** die Entnahme einer vorgegebenen Anzahl von Einzelfasern (10) aus einem Probenbehälter (4) und/oder Entnahmen aus weiteren Probenbehältern (4) mit gleichen oder unterschiedlichen Faserproben programmgesteuert durchgeführt wird.

13. Verfahren nach Anspruch 8 bis 12, **dadurch gekennzeichnet, dass** die Detektion in dem Übergabebereich (30) mit einem optischen Detektionssystem (28), vorzugsweise mit einer Kamera (48) mit angeschlossener Bildverarbeitung (50) durchgeführt wird.

## Claims

1. Device (1) for automatically sampling individual fibers (10) of a fiber sample (6) from a fiber reservoir (8) and for transferring an individual fiber (10) to a subsequent device, in particular a fiber testing device (12), having a control (16) and a transport device (14) for the individual fibers (10), comprising
a fiber reservoir (8) comprising at least one sample container (4) containing the fiber sample (6) in the form of fiber tufts,
a sampling means (18) configured to sample a plurality of individual fibers (10) from the fiber tuft of the fiber sample (6) contained in the sample container (4),
a transport device (14) configured to take over the sampled individual fibers (10) from the sampling device (18) for transferring the individual fibers (10) to the subsequent device (12),
**characterized in that**
the sampling device (18) comprises at least a gripping means (3), a fiber separation means (22) and a detection means (28), wherein the gripping means (3) is configured to sample one or more individual fibers (10) from the sample container (4), and that the fiber separation means (22) is configured to stretch the individual fibers (10) sampled by the gripping means (3) until only a single individual fiber (10) is detected by the detection means (28) and to thereafter release the separated single individual fiber (10) to a clamping means (13) of the transport device (14).

2. Device of claim 1, **characterized in that** the individual fibers (10) adapted to be sampled and held by the gripping means (3) are adapted to be drawn through a plurality of constrictions (24, 26) of the fiber separation means (22) in which the individual fibers (10) can be given a stretched shape.

3. Device of one of claims 1 to 2, **characterized in that** the number of stretched individual fibers (10) can be detected by a detection system (28) of the sampling means (18), wherein the transport device (14) can be activated to take over the separated individual fiber (10) when only a single individual fiber (10) is present in a transfer zone (30) for the transport device (14).

4. Device of one of claims 1 to 3, **characterized in that** the fiber reservoir (8) is formed by a magazine with a plurality of sample containers (4) with identical or different fiber samples (6).

5. Device of one of clams 1 to 4, **characterized in that** at least one gripping means (3) is adapted to be inserted into the fiber sample (6) in the sample container (4) through at least one opening (40), wherein, by actuating the gripping means (3), one or more individual fibers (10) of the fiber sample (6) can be clamped and pulled out of the sample container (4).

6. Device of one of claims 2 to 5, **characterized in that** the constrictions (24, 26) are formed by two pairs of pivot arms (36, 38) provided with recesses (34) adapted to each other, the pivot position of said arms and thus the size of the constrictions (24, 26) between the pairs of pivot arms (36, 38) being adjustable by means of the control (16).

7. Device of one of claims 1 to 6, **characterized in that** the fiber samples (6) in the at least one sample container (4) are adapted to be mixed using compressed air via a blowing nozzle arranged at the at least one opening (40) for the passage of the at least one gripping means (3).

8. Method for automatically sampling individual fibers (10) of a fiber sample (6) from a fiber reservoir (8) and for transferring an individual fiber (10) to a subsequent device, in particular a fiber testing device (12), wherein a plurality of individual fibers (10) is sampled from a fiber sample (6) contained as fiber tufts in a fiber reservoir (8) having at least one sample container (4) and the sampled individual fibers (10) are transferred to the subsequent device (12), **characterized in that**, using a sampling device (18), one or more individual fibers (10) are sampled from the sample container (4), the sampled individual fibers (10) are reduced to a single individual fiber (10) and are stretched, until only a single individual fiber (10) is detected, and the separated individual fiber (10) is then released for transport to the subsequent device (12).

9. Method of claim 8, **characterized in that** the sampled individual fibers (10) are pulled through a plurality of constrictions (24, 26) in which a stretched shape is imparted to the sampled individual fibers (10), wherein the number of the individual fibers (10) present in a transfer zone (30) for transport to the subsequent device (12) is detected, and the take-over of a separated individual fiber (10) for transport is activated when only a single individual fiber (10) is present in the transfer zone (30).

10. Method of claim 8 to 9, **characterized in that** individual fibers (10) of the fiber sample (6) contained in the sample container (4) are sampled by inserting an open gripping means (3) into the fiber sample (6) via at least one opening (40), is closed in the fiber sample (6) and, thereafter, individual fibers (10) are drawn from the fiber sample (6) by means of the gripping means (3).

11. Method of claims 8 to 10, **characterized in that** a plurality of samples are sampled at different sites inside a sample container (4).

12. Method of claims 8 to 11, **characterized in that** sampling a predetermined number of individual fibers (10) from a sample container (4) and/or sampling from further sample containers (4) containing identical or different fiber samples is effected under control by a program.

13. Method of claims 8 to 12, **characterized in that** the detection in the transfer zone (30) is performed using an optical detection system (28), preferably a camera (48) with image processing means (50) connected thereto.

## Revendications

1. Dispositif (1) destiné au prélèvement automatique de fibres individuelles (10) d'un échantillon de fibres (6) à partir d'un réservoir de fibres (8) et au transfert d'une fibre individuelle (10) en direction d'un appareil suivant, en particulier un appareil de contrôle de fibres (12), avec une commande (16) et un système de transport (14) pour les fibres individuelles (10), comprenant
un réservoir de fibres (8), qui présente au moins un conteneur d'échantillons (4) dans lequel l'échantillon de fibres (6) est contenu sous la forme de touffes de fibres,
un système de prélèvement (18) qui est aménagé pour le prélèvement de plusieurs fibres individuelles (10) à partir de la touffe de fibres de l'échantillon de fibres (6) contenue dans le conteneur d'échantillons (4),
un système de transport (14) qui est aménagé pour prendre en charge les fibres individuelles (10) prélevées à partir du système de prélèvement (18) pour la poursuite de l'acheminement des fibres individuelles (10) vers l'appareil (12) suivant,
**caractérisé en ce que** le système de prélèvement (18) présente au moins un système de préhension (3), un système de séparation des fibres (22) et un système de détection (28),
le système de préhension (3) étant aménagé pour prélever une ou plusieurs fibres individuelles (10) à partir du conteneur d'échantillons (4),
et **en ce que** le système de séparation des fibres (22) est aménagé pour étirer les fibres individuelles (10) prélevées par le système de préhension (3) jusqu'à ce que seulement une fibre individuelle (10) unique soit détectée par le système de détection (28) et pour ensuite débloquer la fibre individuelle (10) unique séparée à destination d'un système de serrage (13) du système de transport (14).

2. Dispositif selon la revendication 1, **caractérisé en ce que** les fibres individuelles (10) pouvant être prélevées par le système de préhension (3) et retenues, peuvent être tirées à travers plusieurs rétrécissements (24, 26) du système de séparation des fibres (22) dans lesquels les fibres individuelles (10) peuvent être amenées à adopter une forme étirée.

3. Dispositif selon l'une des revendications 1 à 2, **caractérisé en ce que** le nombre de fibres individuelles (10) étirées peut être détecté par un système de détection (28) du système de prélèvement (18), le système de transport (14) pouvant être activé pour la prise en charge des fibres individuelles (10) séparées quand il ne reste plus qu'une fibre individuelle (10) unique dans une zone de transfert (30) pour le système de transport (14).

4. Dispositif selon l'une des revendications 1 à 3, **caractérisé en ce que** le réservoir de fibres (8) se compose d'un magasin avec plusieurs conteneurs d'échantillons (4) avec des échantillons de fibres (6) identiques ou différents.

5. Dispositif selon l'une des revendications 1 à 4, **caractérisé en ce qu'**au moins un système de préhension (3) peut être introduit à travers une ouverture (40) dans l'échantillon de fibres (6) dans le conteneur d'échantillons (4), une ou plusieurs fibres individuelles (10) provenant de l'échantillon de fibres (6), et pouvant, par l'actionnement du système de préhension (3), être pincées et pouvant être extraites du conteneur d'échantillons (4).

6. Dispositif selon l'une des revendications 2 à 5, **caractérisé en ce que** les rétrécissements (24, 26) sont formés par deux paires de bras pivotants (36, 38), munis d'évidements (34) adaptés les uns aux autres, bras dont la position de pivotement et donc la grandeur des rétrécissements (24, 26) entre les paires de bras pivotants (36, 38) peuvent être réglées par la commande (16) .

7. Dispositif selon l'une des revendications 1 à 6, **caractérisé en ce que** les échantillons de fibres (6) dans le conteneur d'échantillons (4), au moins au nombre de un, peuvent être mélangés intimement avec de l'air comprimé par le biais d'une buse de soufflage disposée sur l'ouverture (40), au moins au nombre de une, destinée au passage du système de préhension (3), au moins au nombre de un.

8. Procédé destiné au prélèvement automatique de fibres individuelles (10) d'un échantillon de fibres (6) à partir d'un réservoir de fibres (8), et au transfert d'une fibre individuelle (10) vers un appareil suivant, en particulier un appareil de contrôle de fibres (12), plusieurs fibres individuelles (10) étant prélevées à partir d'un échantillon de fibres (6) contenu sous forme de touffes de fibres dans un réservoir de fibres (8) avec au moins un conteneur d'échantillons (4), les fibres individuelles (10) prélevées étant transférées vers l'appareil (12) suivant, **caractérisé en ce que**, à l'aide d'un système de prélèvement (18), une ou plusieurs fibres individuelles (10) sont prélevées à partir du conteneur d'échantillons (4), les fibres individuelles (10) prélevées étant réduites à une fibre individuelle (10) unique et étirées jusqu'à jusqu'à ce que seulement une fibre individuelle (10) unique soit détectée, la fibre individuelle (10) unique séparée étant ensuite débloquée pour le transport vers l'appareil (12) suivant.

9. Procédé selon la revendication 8, **caractérisé en ce que** les fibres individuelles (10) prélevées sont tirées à travers plusieurs rétrécissements (24, 26) dans lesquels les fibres individuelles (10) prélevées sont amenées à adopter une forme étirée, le nombre de fibres individuelles (10) situées dans une zone de transfert (30) pour le transport vers l'appareil (12) suivant étant détecté, et la prise en charge d'une fibre individuelle (10) séparée étant activée pour le transport quand il ne reste plus qu'une fibre individuelle (10) unique dans la zone de transfert (30).

10. Procédé selon les revendications 8 à 9, **caractérisé en ce que** des fibres individuelles (10) de l'échantillon de fibres (6) contenu dans le conteneur d'échantillons (4) sont prélevées par le fait qu'un système de préhension (3) est introduit dans l'échantillon de fibres (6) en position ouverte par le biais d'au moins une ouverture (40), par le fait qu'un système de préhension (3) est fermé à l'intérieur de l'échantillon de fibres (6) puis par le fait que des fibres individuelles (10) sont extraites de l'échantillon de fibres (6) avec le système de préhension (3).

11. Procédé selon les revendications 8 à 10, **caractérisé en ce que** plusieurs échantillons sont prélevés en différents endroits à l'intérieur d'un conteneur d'échantillons (4).

12. Procédé selon les revendications 8 à 11, **caractérisé en ce que** le prélèvement d'un nombre prédéfini de fibres individuelles (10) à partir d'un conteneur d'échantillons (4) et/ou des prélèvements à partir d'autres conteneurs d'échantillons (4) avec des échantillons de fibres identiques ou différents est/sont effectué(s) en étant commandé(s) par programme.

13. Procédé selon les revendications 8 à 12, **caractérisé en ce que** la détection dans la zone de transfert (30) est effectuée avec un système de détection (28) optique, de préférence avec une caméra (48), avec ensuite un traitement d'images (50).
